# EUROPEAN PATENT SPECIFICATION

(11) **EP 1 875 907 B1**
(45) Date of publication and mention of the grant of the patent: **14.09.2011**
(21) Application number: 07017631.8
(22) Date of filing: 21.07.2004
(51) Int. Cl.: A61K 31/35, C12Q 1/44

(54) **Use of yessotoxin in the treatment of allergic and asthmatic processes**
Einsatz von Yessotoxin bei der Behandlung allergischer und asthmatischer Leiden
Utilisation de yessotoxin pour le traitement de processus allergiques et asthmatiques

(30) Priority: 25.07.2003 ES 200301773
(43) Date of publication of application: 09.01.2008
(62) Divisional of application: 04742071.6
(73) Proprietor: LABORATORIO CIFGA, S.A., 27001 Lugo (ES)
(72) Inventor: Botana Lopez, Luis Miguel, 15782 Santiago de Compostela (ES); Alfonso Rancaño, Amparo, 15782 Santiago de Compostela (ES); Rodriguez Vieytes, Mercedes, 15782 Santiago de Compostela (ES); Loza Garcia, Maria Isabel, 15782 Santiago de Compostela (ES)
(74) Representative: ABG Patentes, S.L.

(56) References cited:
- EP-A- 1 251 181
- US-A- 5 686 286
- ALFONSO A ET AL: "Yessotoxin, a novel phycotoxin, activates phosphodiesterase activity. Effect of yessotoxin on cAMP levels in human lymphocytes" BIOCHEMICAL PHARMACOLOGY, PERGAMON, OXFORD, GB, vol. 65, no. 2, 15 January 2003 (2003-01-15), pages 193-208, XP002995441 ISSN: 0006-2952

## Description

The present invention describes the therapeutic use of yessotoxin in the treatment of allergy and asthma according to their ability to activate phosphodiesterase enzymes, one of the cellular targets of this toxin.

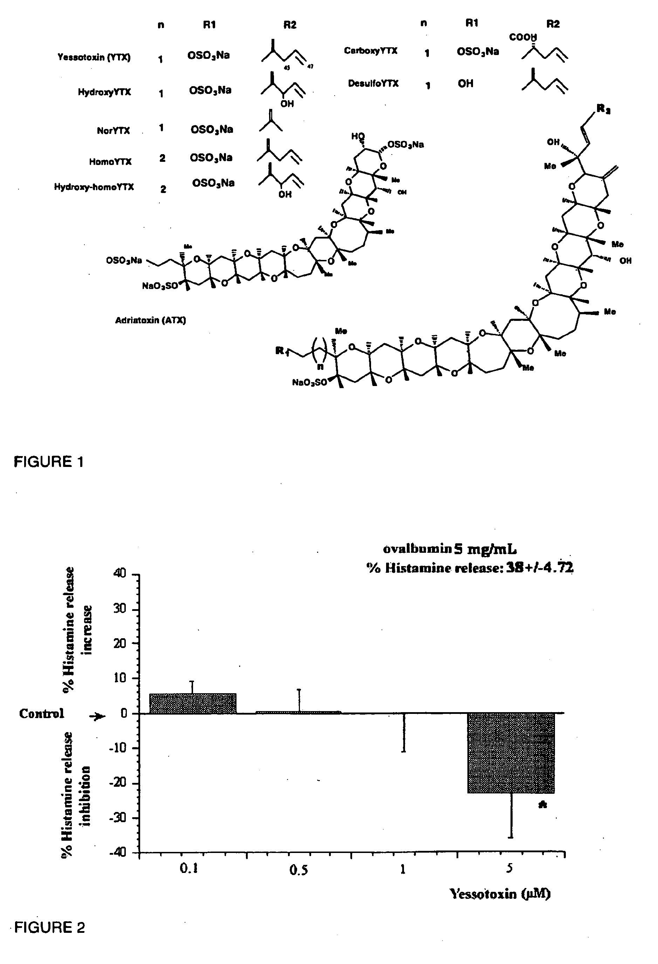

Yessotoxin, hereinafter YTX, is a polycyclic ether produced by microalgae of the *Protoceratium reticulatum* and *Lingolodinium polyedrum* species and originally isolated from the hepatopancreas of *Patinopecten yessoensis* (Murata, M., Kumagai, M. et al., 1987, Tetrahedron Letters, 28, 5869-5872). The structure of YTX is related to that of ciguatera toxins and brevetoxins. As shown in Figure 1, it is a lipophilic molecule made up of eleven rings with ether groups bound to an unsaturated side chain and to two sulfonic esters. Figure 1 further shows some of the natural analogs of YTX which are different in the side chain substituents, though over 50 natural derivatives the structure of which has not yet been identified have recently been described.

YTXs were initially classified within the group of diarrhetic toxins since they are often detected together; however their mechanism of action is different from that of any other toxin. YTX decreases adenosine cyclic phosphate (cAMP) cytosolic levels because it increases the activity of cellular phosphodiesterases by a calcium-dependent mechanism (Alfonso, A., de la Rosa, L.A. et al., 2003, Biochem Pharmacol, 65, 193-208). YTX further increases cytosolic calcium levels by stimulating its entry through a channel located in the cell membrane (De la Rosa, L.A., Alfonso, A. et al., 2001, Biochem. Pharmacol., 61, 827-833; De la Rosa, L.A., Alfonso, A. et al., 2001, Cell Signal, 13, 711-716). The effect on phosphodiesterases has been used to develop sensitive methods for detecting the presence of these toxins in contaminated mollusks described in the main patent application of the present invention, and which have been recently published (Alfonso, A., Vieytes, M.R. et al., 2004, Analytical Biochem., 326, 93-99; Pazos, M.J., Alfonso, A. et al., 2004, Analytical Biochem., 335, 112-118)

On the other hand, some studies have been conducted in order to determine the toxicity of YTX. When it is orally administered to mice, no cases of acute toxicity have been observed; however death of the rodents did occur after an intraperitoneal injection (Tubaro, A., Sosa, S. et al., 2003, Toxicon, 41, 783-92). In this sense, oral administration to a mouse of 10 mg of YTX per Kg of weight does not cause death (Aune, T., Sorby, R. et al., 2002, Toxicon, 40, 77-82). Only some ultrastructural alterations in the myocardium have been observed after oral treatment (Aune, T., Sorby, R. et al., 2002, Toxicon, 40, 77-82; Tubaro, A., Sosa, S. et al., 2003, Toxicon, 41, 783-92), and no accumulative toxic effects occurred after the repeated oral administration of this toxin (Tubaro, A., Sosa, S. et al., 2004, Toxicon, 43, 439-45). After an intraperitoneal administration, YTX was proven to be two times less lethal than okadaic acid, which is the diarrhetic toxin that occurs most frequently and in the largest amount. The main signs observed after a high dose of YTX administered intraperitoneally were restlessness, jumping and finally death. The histological exam showed the presence of myocardial alterations which could be seen only with electron microscopy, with no damage to other organs (Tubaro, A., Sosa, S. et al., 2003, Toxicon, 41, 783-92). Therefore, based on these results, YTX and the derivatives thereof are considered non toxic, even at doses 100 times higher than those which a human being can consume daily.

YTX has a singular structure and its mechanism of action does not seem to be very common, therefore it can be an interesting tool for biological and pharmacological studies. cAMP is a second messenger related to early pathways in the cell signal. Cells regulate cAMP levels by a balance between synthesis, through the activation of adenylate cyclases, and hydrolysis, by activation of phosphodiesterases. There are eleven families of phosphodiesterases with different substrate specificity, sensitivity and location. Modulation of these enzymes is often used to regulate the activity of several types of inflammatory cells; in fact several treatments used in asthma interfere with this pathway. Therefore, it is very interesting to determine the effect that YTX has on inflammatory cells, since the toxin increases interleukin 2 levels in human lymphocytes (Alfonso, A., de la Rosa, L.A. et al., 2003, Biochem Pharmacol, 65, 193-208).

It has recently been observed that YTX is a histamine release inhibitor, which release is activated by immunological stimulus, in mast cells in rats, therefore it is a drug susceptible to being used as an antiallergic or antiasthmatic drug. The role of cAMP in histamine release is not very clear. Generally, an increase of cAMP levels inhibits secretion, however contradictory effects has been observed in the presence of different stimuli (Alfonso, A., Cabado, A.G. et al., 2000, Cell Signal, 12, 343-350; Alm, P.E., 1983, Agents Actions, 13, 132-7; Alm, P.E., 1984, Int Arch Allergy Appl Immunol, 75, 375-8; Teraoka, H., Akiba, H. et al., 1997, Gen Pharmacol, 28, 237-243). In this sense, it has been described that the immunological stimulation is accompanied by an initial increase of cAMP levels which disappears after ten minutes but which is required to activate events associated to secretion since cAMP is part of these initial events (Alm, P.E., 1984, Int Arch Allergy Appl Immunol, 75, 375-8). This fact may explain the results obtained in the presence of YTX, since the toxin prevents the initial increase of cAMP, which in turn inhibits histamine release. Inhibition depends on toxin concentration, as does the decrease in cAMP levels (Alfonso, A., de la Rosa, L.A. et al., 2003, Biochem Pharmacol, 65, 193-208). This fact is not contradictory with the use of phosphodiesterase inhibition to suppress histamine release in the treatment of asthma, since in this case cAMP levels remain high, which also prevents secretion. Therefore, the description of YTX as histamine release modulators is an indication of the pharmacological importance of these molecules for their possible therapeutic application.

Use of phosphodiesterases-activating YTX as inhibitor of the immunological activation of mast cells and basophils.

Immunological activation of mast cells and basophils requires a temporary increase of cAMP. This increase is indispensable for cell response activation (Botana, L.M. and MacGlashan, D.W., 1994, J Leukocyte Biol, 55, 798-804). PDE activation cancels this initial cAMP peak, and therefore prevents cell activation. In the presence of YTX, i.e. with activated PDEs, cell response will be inhibited. The inhibiting effect can be used in antiallergic or antiasthmatic therapeutic strategies, these being two pathologies in which mast cells play a predominant role (Metcalfe, D.D., Baram, D. et al., 1997, Physiol Rev, 77, 1033-1079). The present use describes the quantification of the inhibition that YTX produces on cell activation induced by immunological stimulus in mast cells in rats. Cell response inhibition can be determined according to different protocols described in the literature. One in which the response is quantified according to the histamine released by mast cells in rats into the extracellular medium is set forth below (Alfonso, A., Cabado, A.G. et al., 2000, Br J Pharmacol, 130, 1809-1816; Estévez, M.D., Vieytes, M.R. et al., 1994, Biochem Pharmacol, 47, 591-593).

### EMBODIMENT OF THE INVENTION

a.- The rats are sensitized 15 days before conducting the experiment. Each rat is subcutaneously injected with 1 mL of physiological serum with 150 mg of ovalbumin and 10⁹ *Bordetella pertussis* bacteria.
b.- Mast cells are extracted from the chest and abdomen of a sensitized rat. The two populations are mixed and a cell suspension is obtained.
c.- The cell suspension is preincubated for 10 minutes with various concentrations of YTX and subsequently incubated 10 minutes in the presence of 5 mg/mL of ovalbumin.
d.- The reaction is stopped in cold conditions and the released histamine is separated from the histamine remaining in the cells by means of centrifugation.
e.- The supernatant is removed with the histamine released into the medium and the cells are cleaved with hydrochloric acid and ultrasound in order to release the histamine not sensitive to the action of the stimulus.
f.- Both mediums are deproteinized with trichloroacetic acid.
g.- The histamine is finally quantified, converting it into a fluorescent molecule by reaction in a base medium with o-phthalic dialdehyde. The reaction is stopped with hydrochloric acid and the fluorescence is read at 360 nm excitation and 460 nm emission.

Figure 2 shows the percentage of inhibition of histamine release induced by ovalbumin in the presence of several concentrations of YTX.

### LITERATURE

Alfonso, A., Cabado, A. G., Vieytes, M. R. and Botana, L. M. (2000). "Calcium-pH crosstalks in rat mast cells: cytosolic alkalinization, but not intracellular calcium release, is a sufficient signal for degranulation." Br J Pharmacol 130(8): 1809-1816.

Alfonso, A., Cabado, A. G., Vieytes, M. R. and Botana, L. M. (2000). "Functional compartments in rat mast cells for cAMP and calcium on histamine release." Cell Signal 12(5): 343-350.

Alfonso, A., de la Rosa, L. A., Vieytes, M. R., Yasumoto, T. and Botana, L. M. (2003). "Yessotoxin a novel phycotoxin, activates phosphodiesterase activity. Effect of yessotoxin on cAMP levels in human lymphocytes." Biochem Pharmacol 65: 193-208.

Alfonso, A., Vieytes, M. R., Yasumoto, T. and Botana, L. M. (2004). "A rapid microplate fluorescent method to detect yessotoxins based on their capacity to activate phosphodiesterases." Analytical Biochem. 326: 93-99.

Alm, P. E. (1983). "Sodium fluoride evoked histamine release from mast cells. A study of cyclic AMP levels and effects of catecholamines." Agents Actions 13(2-3):132-7.

Alm, P. E. (1984). "Modulation of mast cell cAMP levels. A regulatory function of calmodulin." Int Arch Allergy Appl Immunol 75(4): 375-8.

Aune, T., Sorby, R., Yasumoto, T., Ramstad, H. and Landsverk, T. (2002). "Comparison of oral and intraperitoneal toxicity of yessotoxin towards mice." Toxicon 40(1): 77-82.

Botana, L. M. and MacGlashan, D. W. (1994). "Differential effects of cAMP-elevating drugs on stimulus-induced cytosolic calcium changes in human basophils." J Leukocyte Biol 55(6): 798-804.

De la Rosa, L. A., Alfonso, A., Vilariño, N., Vieytes, M. R. and Botana, L. M. (2001). "Modulation of cytosolic calcium levels of human lymphocytes by yessotoxin, a novel marine phycotoxin." Biochem. Pharmacol. 61 (7): 827-833.

De la Rosa, L. A., Alfonso, A., Vilariño, N., Vieytes, M. R., Yasumoto, T. and Botana, L. M. (2001). "Maitotoxin-induced calcium entry in human lymphocytes - Modulation by yessotoxin, Ca2+ channel blockers and kinases." Cell Signal 13(10): 711-716.

Estévez, M. D., Vieytes, M. R., Louzao, M. C. and Botana, L. M. (1994). "Effect of okadaic acid on immunologic and non-immunologic histamine release in rat mast cells." Biochem Pharmacol 47(3): 591-593.

Metcalfe, D. D., Baram, D. and Mekori, Y. A. (1997). "Mast cells." Physiol Rev 77(4): 1033-1079.

Murata, M., Kumagai, M., Lee, J. S. and Yasumoto, T. (1987). "Isolation and structure of Yessotoxin, a novel polyether compound implicated in diarrhetic shellfish poisoning." Tetrahedron Letters 28: 5869-5872.

Pazos, M. J., Alfonso, A., Vieytes, M. R., Yasumoto, T., Vieites, J. M. and Botana, L. M. (2004). "Resonant mirror biosensor detection method based on yessotoxin-phosphodiesterase interactions." Analytical Biochem. 335: 112-118.

Teraoka, H., Akiba, H., Takai, R., Taneike, T., Hiraga, T. and Ohga, A. (1997). "Inhibitory effects of caffeine on Ca2+ influx and histamine secretion independent of cAMP in rat peritoneal mast cells." Gen Pharmacol 28(2): 237-243.

Tubaro, A., Sosa, S., Altinier, G., Soranzo, M. R., Satake, M., Della Loggia, R. and Yasumoto, T. (2004). "Short-term oral toxicity of homoyessotoxins, yessotoxin and okadaic acid in mice." Toxicon 43(4): 439-45.

Tubaro, A., Sosa, S., Carbonatto, M., Altinier, G., Vita, F., Melato, M., Satake, M. and Yasumoto, T. (2003). "Oral and intraperitoneal acute toxicity studies of yessotoxin and homoyessotoxins in mice." Toxicon 41(7): 783-92.

## Claims

1. Yessotoxin for use in a method for the treatment of allergic or asthmatic processes.

2. The *in vitro* use of yessotoxin (YTX) as inhibitor of the immunological activation of mast cells.

## Patentansprüche

1. Yessotoxin zur Verwendung in einem Verfahren zur Behandlung allergischer oder asthmatischer Prozesse.

2. In vitro-Verwendung von Yessotoxin (YTX) als Inhibitor für die immunologische Aktivierung von Mastzellen.

## Revendications

1. Yessotoxine pour utilisation dans un procédé pour le traitement de processus allergique ou asthmatique.

2. Utilisation *in vitro* de yessotoxine (YTX) en tant qu'inhibiteur de l'activation immunologique de mastocytes.
